# EUROPEAN PATENT SPECIFICATION

(11) **EP 3 018 604 B1**
(45) Date of publication and mention of the grant of the patent: **11.10.2023**
(21) Application number: 15192572.4
(22) Date of filing: 02.11.2015
(51) Int. Cl.: G16B 30/00, G16B 30/10

(54) **METHOD FOR ASSIGNING TARGET-ENRICHED SEQUENCE READS TO A GENOMIC LOCATION**
VERFAHREN ZUR ZUWEISUNG VON ZIELANGEREICHERTEN SEQUENZAUSLESUNGEN ZU EINER GENOMISCHEN POSITION
PROCÉDÉ D'ATTRIBUTION DE LECTURES DE SÉQUENCES ENRICHIES DE MANIÈRE CIBLÉE À UN EMPLACEMENT GÉNOMIQUE

(30) Priority: 05.11.2014 US 201414533743
(43) Date of publication of application: 11.05.2016
(73) Proprietor: Agilent Technologies, Inc., Santa Clara, CA 95051 (US)
(72) Inventor: LE COCQ, Christian A., Santa Clara, CA 95051-7201 (US); ISAKSSON, Magnus, Santa Clara, CA 95051-7201 (US); LNU, Ashutosh, Santa Clara, CA 95051-7201 (US); FORSMARK, Linus, Santa Clara, CA 95051-7201 (US)
(74) Representative: Grünecker Patent- und Rechtsanwälte PartG mbB

(56) References cited:
- WO-A1-2011/067378
- R. BOTTCHER ET AL: "Using a priori knowledge to align sequencing reads to their exact genomic position", NUCLEIC ACIDS RESEARCH, vol. 40, no. 16, 11 May 2012 (2012-05-11), pages e125-e125, XP055256886, GB ISSN: 0305-1048, DOI: 10.1093/nar/gks393
- K. BODI ET AL: "Comparison of Commercially Available Target Enrichment Methods for Next-Generation Sequencing", JOURNAL OF BIOMOLECULAR TECHNIQUES, 1 July 2013 (2013-07-01), XP055256899, US ISSN: 1524-0215, DOI: 10.7171/jbt.13-2402-002
- COLE TRAPNELL ET AL: "How to map billions of short reads onto genomes", NATURE BIOTECHNOLOGY, vol. 27, no. 5, 1 May 2009 (2009-05-01), pages 455-457, XP055110041, ISSN: 1087-0156, DOI: 10.1038/nbt0509-455
- Ellen Knierim ET AL: "Systematic Comparison of Three Methods for Fragmentation of Long-Range PCR Products for Next Generation Sequencing", PLoS ONE, vol. 6, no. 11, 30 November 2011 (2011-11-30), page e28240, XP055236211, DOI: 10.1371/journal.pone.0028240

## Description

### BACKGROUND

Target enrichment methods are used in the fields of diagnostics and clinical research in order to accelerate Next Generation Sequencing (NGS) workflows. Such methods isolate from the sample DNA only the fragments that match probes designed to target a suite of genomic regions of interest.

In many cases, the first processing step in extracting diagnostic information from NGS data is to tag each sequence read (or pair of reads) with its likely genomic location. General purpose aligners that are available for this purpose, such as the Burrows-Wheeler Aligner (BWA), are programs that try to find for each read (or read pair) the best match across the whole genome. However, programs like BWA use information provided only by the sequence of each read and by the whole genome reference data to align the sequences. Thus general purpose aligners do not integrate the target enrichment parameters in the matching algorithm when sequence reads are obtained from samples enriched using a target enrichment panel, resulting in longer processing times and reduced accuracy of tagging.
Böttcher, R. et al. (Nucl. Acids Res., 2012, vol. 40, no. 16, e125) discloses a method of accelerating sequence alignments using a priori knowledge to align sequencing reads to their exact genomic position, while the present invention starts from samples for which no prior knowledge about sequence reads is available

### SUMMARY

Provided herein is a computer-implemented method for assigning a sequence read to a genomic location, the method including: a) accessing a file containing a sequence read, wherein the sequence read is obtained from a nucleic acid sample that has been enriched by hybridization to a plurality of capture sequences; and b) assigning the sequence read to a genomic location by: i) identifying a capture sequence as being a match with the sequence read if the sequence read contains one or more subsequences of the capture sequence; ii) calculating, using a computer, a score indicating the degree of sequence similarity between each of the matched capture sequences and the sequence read; and iii) assigning the sequence read to the genomic location if the calculated score for a matched capture sequence is above a threshold, the method being further specified by the appended claims.

In certain embodiments, the identifying step i) includes identifying one or more of the capture sequences as being a match with the sequence read if a terminal region of the sequence read contains one or more subsequences of the capture sequences. In such embodiments, the terminal region may be in the range of 10 bp to 50 bp from an end of the sequence read. In certain embodiments, the one or more subsequences are in the range of 5 bp to 15 bp in length.

In any of the above embodiments, the one or more subsequences of the capture sequence is selected from between 4 to 20 subsequences of the capture sequence.

In any of the above embodiments, the subsequences are tiled across the entire capture sequence.

In any of the above embodiments, the calculated score is based on the length of sequence identity between the matched capture sequence and the sequence read, the string edit distance between the matched capture sequence and the sequence read, the position within the sequence read of each of the mismatches, or a combination thereof.

In any of the above embodiments, step i) further includes generating a data structure, wherein the capture sequences are stored in the data structure as values mapped by sequence keys containing subsequences of the capture sequences, and the identifying step includes identifying one or more of the capture sequences as being a match with the sequence read if the sequence read contains one or more sequence keys.

In the inventive method as claimed, the sequence read is a paired-end sequence read.

In any of the above embodiments, the enriched sample includes amplified copies of fragmented genomic nucleic acids, wherein the fragmented genomic nucleic acids are enriched by hybridization to the plurality of capture sequences. In such embodiments, the fragmented genomic nucleic acids are fragmented by enzymatically cleaving genomic nucleic acids at predetermined sites.

In any of the above embodiments, the plurality of capture sequences that hybridize to an end of the nucleic acids.

In any of the above embodiments, the assigning step b) further includes discarding a sequence read if the sequence read does not contain any subsequences of the capture sequences.

In any of the above embodiments, the method is performed on a plurality of sequence reads, thereby assigning a plurality of sequence reads to genomic locations.

In any of the above embodiments, the assigning step b) further includes: iv) identifying a matched capture sequence having the highest calculated score among all of the matched capture sequences as being the best match; and v) assigning the sequence read to the genomic location by adding the sequence read to a set of unique sequence reads matching the best matched capture sequence, wherein each unique sequence read in the set contains a subsequence identical to a subsequence of all the other sequence reads in the set. In such embodiments, the subsequence identical to a subsequence of all the other sequence reads in the set may be a barcode sequence. In certain embodiments, the method further includes counting the number of sets of unique sequence reads assigned to a capture sequence.

In any of the above embodiments, the capture sequences include from 10² to 10⁸ distinct sequences.

Also provided herein is a method for assigning a sequence read to a genomic location, involving: a) inputting a set of capture sequences used to enrich a nucleic acid sample by hybridization to a plurality of capture sequences in the set into a computer system containing a sequence read assignment program, wherein the sequence read assignment program includes instructions of a) to b) of the method described above; b) inputting a file containing the sequence read into the sequence read assignment program; and c) executing the sequence read assignment program.

Also provided herein is a computer readable storage medium containing a sequence read assignment program including instructions for: a) accessing a file containing a sequence read, wherein the sequence read is obtained from a nucleic acid sample that has been enriched by hybridization to a plurality of capture sequences; and b) assigning the sequence read to a genomic location by: i) identifying a capture sequence as being a match with the sequence read if the sequence read contains one or more subsequences of the capture sequence; ii) calculating, using a computer, a score indicating the degree of sequence similarity between each of the matched capture sequences and the sequence read; and iii) assigning the sequence read to the genomic location if the calculated score for a matched capture sequence is above a threshold, the method being further specified by the appended claims.

These and other embodiments and implementations are described in greater detail below.

### BRIEF DESCRIPTION OF THE FIGURES

The skilled artisan will understand that the drawings, described below, are for illustration purposes only. The drawings are not intended to limit the scope of the present teachings in any way.
FIG. 1 is a flow chart illustrating one embodiment of the present disclosure.
FIG. 2 is a flow chart illustrating another embodiment of the present disclosure.

### DEFINITIONS

Unless defined otherwise, all technical and scientific terms used herein have the same meaning as commonly understood by one of ordinary skill in the art to which this disclosure belongs.

The terms "polynucleotide", "nucleotide", "nucleotide sequence", "nucleic acid", "nucleic acid molecule", "nucleic acid sequence" and "oligonucleotide" are used interchangeably, and can also include plurals of each respectively depending on the context in which the terms are utilized. They refer to a polymeric form of nucleotides of any length, either deoxyribonucleotides (DNA) or ribonucleotides (RNA), or analogs thereof. Polynucleotides may have any three-dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA (tRNA), ribosomal RNA, ribozymes, small interfering RNA, (siRNA), microRNA (miRNA), small nuclear RNA (snRNA), cDNA, recombinant polynucleotides, branched polynucleotides, plasmids, vectors, isolated DNA (A, B and Z structures) of any sequence, PNA, locked nucleic acid (LNA), TNA (treose nucleic acid), isolated RNA of any sequence, nucleic acid probes, and primers. LNA, often referred to as inaccessible RNA, is a modified RNA nucleotide. The ribose moiety of an LNA nucleotide is modified with an extra bridge connecting the 2' and 4' carbons. The bridge "locks" the ribose in the 3'-endo structural conformation, which is often found in the A-form of DNA or RNA, which can significantly improve thermal stability.

Depending on the context, a nucleotide or a nucleotide sequence may refer to the sequence information contained in the nucleotide or nucleic acid molecule, i.e., the type of nucleotide or the sequence of the type of the nucleotides that make up a nucleic acid molecule.

The term "sequencing," as used herein, refers to a method by which the identity of at least 10 consecutive nucleotides (e.g., the identity of at least 20, at least 50, at least 100 or at least 200 or more consecutive nucleotides) of a polynucleotide are obtained.

The term "next-generation sequencing" refers to the so-called parallelized sequencing-by-synthesis or sequencing-by-ligation platforms currently employed by Illumina, Life Technologies, and Roche, etc. Next-generation sequencing methods may also include nanopore sequencing methods or electronic-detection based methods such as Ion Torrent technology commercialized by Life Technologies.

The term "sequence reads" refers to the output of a sequencing run. Sequence reads are represented by a string of nucleotides. Sequence reads may be accompanied by metrics about the quality of the sequence. For example, each nucleotide in a sequence read may be associated with the confidence of the base call, i.e., a determination of whether a nucleotide is a G, A, T or C, for that position.

The term "amplifying" as used herein refers to generating one or more copies of a target nucleic acid, using the target nucleic acid as a template.

"Hybridization" refers to a reaction in which one or more polynucleotides react to form a complex that is stabilized via hydrogen bonding between the bases of the nucleotide residues. The hydrogen bonding may occur by Watson-Crick base pairing, Hoogstein binding, or in any other sequence-specific manner. The complex may comprise two strands forming a duplex structure, three or more strands forming a multi-stranded complex, a single self-hybridizing strand, or any combination of these.

As is known to one skilled in the art, hybridization can be performed under conditions of various stringency. Suitable hybridization conditions are such that the recognition interaction between a capture sequence and a target nucleic acid is both sufficiently specific and sufficiently stable. Conditions that increase the stringency of a hybridization reaction are widely known and published in the art. See, for example, Green, et al., (2012), infra.

A "plurality" contains at least 2 members. In certain cases, a plurality may have at least 10, at least 100, at least 1000, at least 10,000, at least 100,000, at least 10⁶, at least 10⁷, at least 10⁸ or at least 10⁹ or more members.

The term "reference sequence" refers to a known sequence, e.g., a sequence from a public or in-house database, to which a candidate sequence can be compared. The reference sequence may be a reference genomic sequence.

The terms "genomic location," or "genomic region," as used herein, are used interchangeably to refer to a region of a genome, e.g., an animal or plant genome such as the genome of a human, monkey, rat, fish or insect or plant.

The terms "assigning," "associating," "tagging," in reference to a sequence read, are used interchangeably herein to refer to a process of annotating a sequence read with one or more sources from which the sequence read is likely to have been derived. The source of the sequence read may be represented by a capture sequence used to enrich the nucleic acid sample from which the sequence read was derived, or may be represented by a location of a reference genome, etc.

The term "enriched sample" refers to a sample that contains fragments of genomic DNA that have been isolated from the remainder of the genome. Enriched fragments can be of any length depending on the fragmentation method used. In certain embodiments, the fragments may be in the range of 100 bp to 3 kb in length, e.g., 100 bp to 2500 bp in length, including 200 bp to 1000 bp in length, although fragments outside of this range may be used. Depending on how the fragmentation and/or enriching is done, for any one enriched region, the ends of the fragment molecules may be the same or different.

The term "enriching," with respect to a genome, refers to the separation of one or more regions of a genome from the remainder of the genome to produce a product that is isolated from the remainder of the genome. Enriching may be done using a variety of methods including those described in, e.g., Hedges et al (Comparison of three targeted enrichment strategies on the SOLiD sequencing platform. PLoS One 2011 6: e18595) and Shearer et al (Solution-based targeted genomic enrichment for precious DNA samples BMC Biotechnol. 2012 12: 20*).*

A "capture sequence," as used herein, refers to a nucleotide sequence that can hybridize to target nucleic acids, e.g., genomic fragments containing sequences from a genomic location of interest, and may be used to enrich the target nucleic acids in the sample relative to other nucleic acids that are not of interest. A capture sequence may contain a nucleotide sequence that is complementary to the target nucleic acid.

As used herein, a "subsequence" refers to a sequence of nucleotides contained within a longer nucleotide sequence. Thus, a subsequence of a nucleotide sequence has a nucleotide sequence identical to at least a portion of the nucleotide sequence, or its reverse complement. The subsequence of a nucleotide sequence may be shorter than the nucleotide sequence by one nt (nucleotides) or more, e.g., 2 nt or more, including 3 nt or more, 4 nt or more, 5 nt or more, 6 nt or more, 7 nt or more, 8 nt or more, 9 nt or more, 10 nt or more, 15 nt or more, 20 nt or more, 25 nt or more, 30 nt or more, or 50 nt or more. The subsequence of a nucleotide sequence may have a length of between 3 nt up to 1 nt less than the length of the nucleotide sequence, e.g., a length of between 4 nt up to 3 nt less than the length of the nucleotide sequence, including a length of between 5 nt up to 5 nt less than the length of the nucleotide sequence, a length of between 6 nt up to 10 nt less than the length of the nucleotide sequence.

The term "data structure" refers to a way of organizing data that facilitates use of the data. A data structure may include a table, such as a hash table, a database, an array, a set, a graph, etc.

As used herein, a "sequence key," in the context of a table, refers to a sequence of elements, e.g., a sequence of nucleotides, that is used to locate a value associated with the sequence key in the table. Thus, a table storing a nucleotide sequence can map a sequence key derived from the nucleotide sequence to the nucleotide sequence. For example, the sequence key may be a subsequence of the nucleotide sequence.

### DETAILED DESCRIPTION

The scope of the present teachings will be limited only by the appended claims.

The section headings used herein are for organizational purposes

Where a range of values is provided, it is understood that each intervening value, to the tenth of the unit of the lower limit unless the context clearly dictates otherwise, between the upper and lower limit of that range and any other stated or intervening value in that stated range is encompassed within the present disclosure.

The citation of any publication is for its disclosure prior to the filing date and should not be construed as an admission that the present claims are not entitled to antedate such publication by virtue of prior invention. Further, the dates of publication provided can be different from the actual publication dates which can need to be independently confirmed.

It must be noted that as used herein and in the appended claims, the singular forms "a," "an," and "the" include plural referents unless the context clearly dictates otherwise. It is further noted that the claims can be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which can be readily separated from or combined with the features of any of the other several embodiments. Any recited method can be carried out in the order of events recited or in any other order which is logically possible.

The practice of various embodiments of the disclosure employs, unless otherwise indicated, conventional techniques of biochemistry, chemistry, molecular biology, genomics and recombinant DNA, which are within the skill of the art. See, e.g., Green and Sambrook, MOLECULAR CLONING: A LABORATORY MANUAL, 4th edition (2012); SHORT PROTOCOLS IN MOLECULAR BIOLOGY (F. M. Ausubel, et al. eds., (1995)); the series METHODS IN ENZYMOLOGY (Academic Press, Inc.); and PCR 2: A PRACTICAL APPROACH (M. J. MacPherson, B. D. Hames and G. R. Taylor eds. (1995)).

Provided herein is a computer-implemented method of assigning a sequence read to a genomic location, i.e., a method of identifying the location within a genome from which a nucleotide sequence identified in a sequencing run is derived. In certain embodiments, the subject method is employed to assign to a genomic location a sequence read that is obtained from a nucleic acid sample that has been enriched by hybridization to a plurality of capture sequences that target multiple genomic locations of interest. When sequence reads are obtained using a target enrichment process, the biochemical method of enrichment constrains the possible genomic locations that can be the source of a read (or read pair). This reduces the complexity of the matching space considerably, i.e. from ~3×10⁹ locations (e.g. the entire human genome) to at most ~2.5×10⁶ locations (e.g. the human exome), and for many target enrichment panels that contain a plurality of capture sequences, just a few thousand possible locations.

Also provided herein is a sequence read assignment program including instructions for performing the subject method, a computer readable storage medium containing the sequence read assignment program, and a method for performing the subject method using the sequence read assignment program on a computer system. Further details of the present disclosure are described below.

### METHOD FOR ASSIGNING A SEQUENCE READ TO A GENOMIC LOCATION

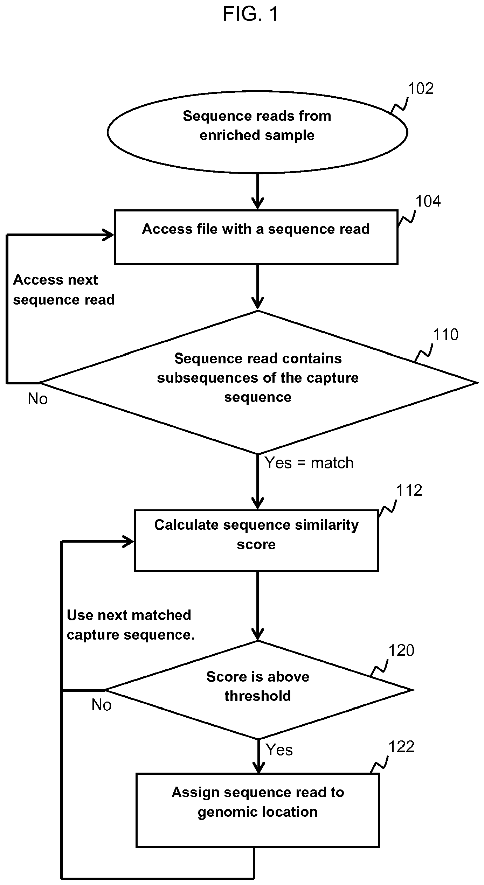

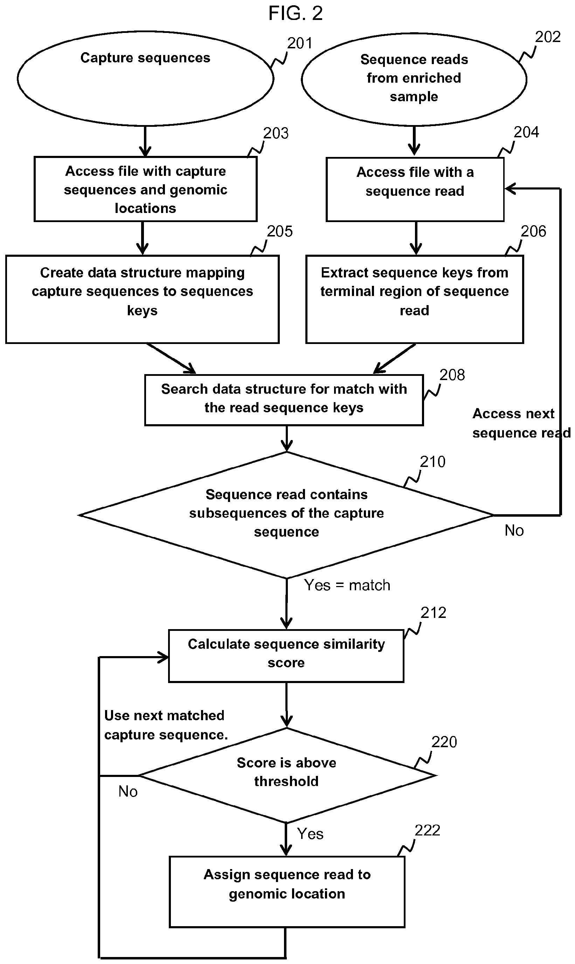

As summarized above, aspects of the present disclosure involve a computer-implemented method for assigning a sequence read to a genomic location. Aspects of the method includes a) accessing a file containing a sequence read, wherein the sequence read is obtained from a nucleic acid sample that has been enriched by hybridization to a plurality of capture sequences; and b) assigning the sequence read to a genomic location by: i) identifying a capture sequence as being a match with the sequence read if the sequence read contains one or more subsequences of the capture sequence; ii) calculating, using a computer, a score indicating the degree of sequence similarity between each of the matched capture sequences and the sequence read; and iii) assigning the sequence read to the genomic location if the calculated score for a matched capture sequence is above a threshold. An embodiment of the present disclosure will be described in further detail with reference to Figs. 1 and 2.

As shown in Fig.1, the present method may be performed on sequence reads (102) obtained from a sample enriched by hybridization to a plurality of capture sequences. In certain embodiments, the sequence reads (102) are obtained from a sample that has been enriched for a particular genomic region, i.e., a sample that contains fragments of genomic DNA that correspond to a particular genomic region, where the fragments have been enriched from fragmented total genomic DNA. In some cases, the enriched genomic region may contain a gene that has a mutation that is associated with one or more cancers, e.g., breast cancer, melanoma, renal cancer, endometrial cancer, ovarian cancer, pancreatic cancer, leukemia, colorectal cancer, prostate cancer, mesothelioma, glioma, medullobastoma, polycythemia, lymphoma, sarcoma or multiple myeloma, etc. (see, e.g., Chial Proto-oncogenes to oncogenes to cancer. Nature Education 2008 1:1). Genes of interest include, but are not limited to, PIK3CA, NRAS, KRAS, JAK2, HRAS, FGFR3, FGFR1, EGFR, CDK4, BRAF, RET, PGDFRA, KIT and ERBB2. In particular cases the sample may contain fragments of genomic DNA that correspond to multiple different genomic regions (e.g., several different regions, e.g., at least 2, at least 5, at least 10, at least 50, at least 100 or at least 1,000 or more different, non-overlapping, regions) that have been enriched, where each region may correspond to a gene, e.g., an oncogene.

The enriched genomic region may be enriched from an initial genomic sample using any convenient method, e.g., using hybridization to an oligonucleotide capture probe or using a ligation-based method. In some embodiments, the genomic region may be enriched by hybridization in solution to one or more biotinylated oligonucleotide capture probes (which, in certain cases, may be RNA oligonucleotides) that may be from 20 to 200 nt in length, e.g., 100 to 150 nt in length, to capture regions of interest. In these embodiments, after capture, duplexes containing fragments of genomic DNA that hybridize to the oligonucleotides may be isolated from other fragments using, e.g., streptavidin beads. In other embodiments, the region of interest may be enriched using the method described by Dahl et al (Multiplex amplification enabled by selective circularization of large sets of genomic DNA fragments. Nucleic Acids Res. 2005 33: e71). In this method, a genomic sample may be fragmented using one or more restriction enzymes and denatured. In this method, a capture probe library or panel is hybridized to the targeted fragments. Each capture probe is an oligonucleotide designed to hybridize to both ends of a targeted DNA restriction fragment, thereby guiding the targeted fragments to form circular DNA molecules. The circular molecules are then closed by ligation, a very precise reaction that ensures that only perfectly hybridized fragments are circularized. Next, the circular DNA targets are amplified. Other enrichment methods may be described in, e.g., Hedges et al (Comparison of three targeted enrichment strategies on the SOLiD sequencing platform. PLoS One 2011 6: e18595) and Shearer et al (Solution-based targeted genomic enrichment for precious DNA samples BMC Biotechnol. 2012 12: 20*).*

In some instances, each capture probe contains a capture sequence that is designed to hybridize to an end of a nucleic acid, e.g. a targeted DNA restriction fragment. In such cases, a nucleic acid sample, e.g., a fragmented genomic sample, may be enriched for target fragments, e.g., target genomic restriction fragments, by a plurality of capture sequences that hybridize to an end of nucleic acids in the sample. In certain embodiments, the capture sequences contain sequences that hybridize to genomic sites that are expected to be at the ends of genomic restriction fragments produced by enzymatically cleaving a genomic sample. The length of the capture sequence at each end of a capture probe may be in the range of 10 bp to 50 bp, e.g., 12 bp to 40 bp, including 15 bp to 30 bp, 17 bp to 25 bp, or 18 bp to 22 bp. In certain instances the capture sequence at each end of a capture probe is about 20 bp long. In certain embodiments the capture sequences in a capture probe library or target enrichment panel represent from about 10 to about 10¹⁰, e.g., about 50 to about 10⁹, including about 100 to about 10⁸, about 10³ to about 10⁸, or about 10⁴ to about 10⁸ distinct sequences.

The capture probe may contain additional functional sequences and moieties, e.g., primer binding sites, bar code sequences, binding members such as biotin, etc., that facilitate enrichment, sequencing, and/or analysis of target nucleic acids from a sample. Thus, the capture probe may contain a method-specific sequencing motif that is incorporated during circularization. In some cases, the capture probes are biotinylated and the targeted fragments can be retrieved using streptavidin beads.

The capture probe may also contain an identification tag that distinguishes each individual nucleic acid molecule from each other. In other words, the identification tag of a capture probe uniquely identifies the capture probe from all the other capture probes that are present in a solution, e.g, a solution in which capture probes are hybridized to sample nucleic acids for enrichment. In certain embodiments the identification tag may be a barcode oligonucleotide sequence that is a subsequence of the capture probe oligonucleotide. Thus, the barcode sequence allows for subsequent correlation of a sequence read with a population of nucleic acids, e.g., amplification products of enriched genomic fragments, from which the sequence read is derived.

The genomic DNA may be isolated from any organism. The organism may be a prokaryote or a eukaryote. In certain cases, the organism may be a plant, e.g., *Arabidopsis* or maize, or an animal, including reptiles, mammals, birds, fish, and amphibians. In some cases, the initial genomic sample may be isolated from a human or rodent, such as a mouse or a rat. In exemplary embodiments, the initial genomic sample may contain genomic DNA from a mammalian cell, such as, a human, mouse, rat, or monkey cell. Methods of preparing genomic DNA for analysis is routine and known in the art, such as those described by Ausubel, F. M. et al., (1995), supra, and Green et al., (2012), supra. The initial genomic sample may contain genomic DNA or an amplified version thereof (e.g., genomic DNA amplified by a whole genome amplification method using the methods of Lage et al (Genome Res. 2003 13: 294-307), Zong et al (Science. 2012 338 :1622-1626), or published patent application US20040241658, for example). Fragments may be made by fragmenting a genome using physical methods (e.g., sonication, nebulization, or shearing), chemically, enzymatically (e.g., using a rare-cutting restriction enzyme) or using a transposable element (see, e.g., Caruccio Methods Mol. Biol. 2011 733: 241-55; Kaper et al, Proc. Natl. Acad. Sci. 2013 110: 5552-7; Marine et al, Appl. Environ. Microbiol. 2011 77: 8071-9 and US20100120098).

In certain embodiments, the enriched sample comprises fragmented genomic nucleic acids that are enriched by hybridization to a plurality of capture sequences. In such instances, the fragmented genomic nucleic acids may be genomic nucleic acids enzymatically cleaved at predetermined sites based on the restriction enzymes used.

The sample may be made from cultured cells or cells of a clinical sample, e.g., a tissue biopsy, scrape or lavage or cells of a forensic sample (i.e., cells of a sample collected at a crime scene). In particular embodiments, the nucleic acid sample may be obtained from a biological sample such as cells, tissues, bodily fluids, and stool. Bodily fluids of interest include but are not limited to, blood, serum, plasma, saliva, mucous, phlegm, cerebral spinal fluid, pleural fluid, tears, lactal duct fluid, lymph, sputum, cerebrospinal fluid, synovial fluid, urine, amniotic fluid, and semen. In particular embodiments, a sample may be obtained from a subject, e.g., a human, and it may be processed prior to use in the present method. For example, the nucleic acid may be extracted from the sample prior to use, methods for which are known. In particular embodiments, the genomic sample may be from a formalin fixed paraffin embedded (FFPE) sample.

Depending on which method is implemented, the initial sample (i.e., prior to enrichment) may contain fragments of genomic DNA that are already adaptor-ligated. In other embodiments, the fragments may be ligated to an adaptor after they have been enriched.

In some cases, samples may be pooled. In these embodiments, the fragments may have a molecular barcode to indicate their source. In some embodiments the DNA being analyzed may be derived from a single source (e.g., a single organism, virus, tissue, cell, subject, etc.), whereas in other embodiments, the nucleic acid sample may be a pool of nucleic acids extracted from a plurality of sources (e.g., a pool of nucleic acids from a plurality of organisms, tissues, cells, subjects, etc.), where by "plurality" is meant two or more. As such, in certain embodiments, the sample can contain nucleic acids from 2 or more sources, 3 or more sources, 5 or more sources, 10 or more sources, 50 or more sources, 100 or more sources, 500 or more sources, 1000 or more sources, 5000 or more sources, up to and including about 10,000 or more sources. Molecular barcodes may allow the sequences from different sources to be distinguished after they are analyzed, as described above.

After an enriched sample has been obtained, it is amplified and sequenced. In certain embodiments, the fragments are amplified using primers that are compatible with use in, e.g., Illumina's reversible terminator method, Roche's pyrosequencing method (454), Life Technologies' sequencing by ligation (the SOLiD platform) or Life Technologies' Ion Torrent platform. Examples of such methods are described in the following references: Margulies et al (Nature 2005 437: 376-80); Ronaghi et al (Analytical Biochemistry 1996 242: 84-9); Shendure et al (Science 2005 309: 1728-32); Imelfort et al (Brief Bioinform. 2009 10:609-18); Fox et al (Methods Mol Biol. 2009;553:79-108); Appleby et al (Methods Mol Biol. 2009;513:19-39) and Morozova et al (Genomics. 2008 92:255-64), which can be used as reference for the general descriptions of the methods and the particular steps of the methods, including all starting products, reagents, and final products for each of the steps.

In one embodiment, the isolated product may be sequenced using nanopore sequencing (e.g. as described in Soni et al. 2007 Clin. Chem. 53: 1996-2001, or as described by Oxford Nanopore Technologies). Nanopore sequencing is a single-molecule sequencing technology whereby a single molecule of DNA is sequenced directly as it passes through a nanopore. A nanopore is a small hole, of the order of 1 nanometer in diameter. Immersion of a nanopore in a conducting fluid and application of a potential (voltage) across it results in a slight electrical current due to conduction of ions through the nanopore. The amount of current which flows is sensitive to the size and shape of the nanopore. As a DNA molecule passes through a nanopore, each nucleotide on the DNA molecule obstructs the nanopore to a different degree, changing the magnitude of the current through the nanopore in different degrees. Thus, this change in the current as the DNA molecule passes through the nanopore represents a reading of the DNA sequence. Nanopore sequencing technology is disclosed in U.S. Pat. Nos. 5,795,782, 6,015,714, 6,627,067, 7,238,485 and 7,258,838 and U.S. Pat Appln Nos. 2006003171 and 20090029477.

In some embodiments, the sequencing may produce, for each enriched region at least 100, at least 1,000, at least 10,000 up to 100,000 or more sequence reads (**102**). The length of the sequence reads (**102**) may vary greatly depending on, for example, the platform used. In some embodiments, the length of sequence reads (**102**) may be in the region of 30 to 800 bases and, in some cases, may include paired end sequence reads.

As shown in Fig. 1, aspects of the present disclosure include a computer-implemented method for assigning a sequence read to a genomic location including, among other steps, accessing a file (**104**) that contains a sequence read obtained, e.g., by sequencing as described above, from a nucleic acid sample that has been enriched by hybridization to a plurality of capture sequences. The file containing the sequence read may be present on the same computer that is implementing the subject method, or may be present on a distinct computer, e.g., on a remote server, that is configured to communicate with the computer implementing the subject method. In certain embodiments, accessing the file (**104**) involves opening the file so that contents of the file, e.g., sequence reads (**102**) from a sequencing run, may be read and assigned to a genomic location. In some embodiments, a sequence read to be assigned to a genomic location is read into working memory.

After accessing a file that contains a sequence read (**104**), the present method of assigning a sequence read to a genomic location includes, among other steps, identifying a capture sequence as being a match with the sequence read if the sequence read contains one or more subsequences of the capture sequence (**110**). A match between a first nucleotide sequence and a second nucleotide sequence occurs when one nucleotide sequence contains a sequence, i.e., a subsequence, that is identical to a subsequence, or the reverse complement thereof, of the second nucleotide sequence. In other words, a first nucleotide sequence and a second nucleotide sequence are a match if the first nucleotide sequence contains a subsequence that has the same length and the identical order in the 5' to 3' direction of nucleotides (e.g., A, G, T or C for DNA) as the length and sequence of nucleotides, respectively, of a subsequence, or reverse complement thereof, of the second nucleotide sequence. Any suitable method may be used to determine the identity between two nucleotide sequences/subsequences.

Thus, in certain embodiments, a capture sequence is identified as being a match with the sequence read if the sequence read contains one or more subsequences that are identical to a subsequence of the capture sequence (**110**). In certain embodiments, the one or more subsequences of the capture sequence that matches with a sequence read are in the range of 4 bp to 20 bp, e.g., 4 to 18 bp, including 5 to 15 bp, or 6 to 10 bp in length. In certain embodiments, the capture sequence is divided into between, e.g., 3 to 30, including 3 to 25, 4 to 20, 4 to 10, or 4 to 8 subsequences, one or more of which are contained in the sequence read if the sequence read matches the capture sequence. Thus, in certain embodiments, one or more subsequences of the capture sequence are compared with a subsequence of the capture sequence selected from between, e.g., 3 to 30, including 3 to 25, 4 to 20, 4 to 10, or 4 to 8 subsequences of the capture sequence.

In certain embodiments, the subsequences of the capture sequence span the entire capture sequences. In such instances, all the subsequences combined comprise the entire capture sequence. In some embodiments, the subsequences of the capture sequence are tiled across the entire capture sequence, such that none of the subsequences overlap with any other subsequence of the capture sequence. In some embodiments, the subsequences of the capture sequence span the entire capture sequence wherein the subsequence are distributed along the capture sequence in a sliding window, such that consecutive subsequences are offset from one another by a number in the range from one nucleotide to the length of the subsequence.

In certain embodiments, a capture sequence is identified as being a match with a sequence read if a terminal region of the sequence read contains one or more subsequences of the capture sequence. In certain embodiments, the terminal region of the sequence read that contains one or more subsequences of the capture sequence is in the range of 5 bp to 100 bp, e.g., 7 bp to 80 bp, including 10 bp to 50 bp, 12 bp to 40 bp, or 15 bp to 30 bp, from an end of the sequence read. In certain embodiments, a capture sequence is identified as being a match with a sequence read if a terminal region of the sequence read contains one or more subsequences that are identical to a subsequence of the capture sequence.

Any suitable method may be used to determine if a sequence read contains one or more subsequences that are identical to a subsequence of a capture sequence. Fig. 2 shows an implementation of the present method wherein the step of assigning the sequence read to a genomic location includes generating a data structure (**205**). The data structure may be generated by first accessing a file (**203**) containing the capture sequences (**201**) used to enrich the target nucleic acids, and storing the capture sequences in the data structure as values mapped by sequence keys containing subsequences of the capture sequences (**205**). Read sequence keys containing subsequences of the sequence read may be extracted from the sequence read, e.g., a terminal region of the sequence read (**206**). In such instances, the identifying step includes searching the data structure containing the capture sequences using the read sequence keys (**208**), and identifying one or more of the capture sequences as being a match with the sequence read if the sequence read contains one or more sequence keys (**210**).

In certain embodiments, no capture sequence may match a sequence read if the sequence read does not contain any subsequence of the capture sequence. In such instances, the method may be repeated by using a different set of subsequences of the capture sequence, or the method may be repeated using the same set of subsequences of the capture sequence but with a less stringent criteria for matching, i.e., less than 100% identity between the capture sequence subsequence and the sequence read subsequence may qualify as a match between the capture sequence and the sequence read. In certain embodiments, a sequence read for which no match is identified with a capture sequence is discarded from further analysis.

As shown in Figs. 1 and 2, after a match between the sequence read and a capture sequence is identified (**110**, **210**), a score indicating the degree of sequence similarity between each of the matched capture sequences and the sequence read is calculated (**112**, **212**). Thus, a further aspect of the present disclosure includes calculating, using a computer, a score indicating the degree of sequence similarity between each of the matched capture sequences and the sequence read (**112**, **212**). As used herein, "sequence similarity" or "similarity" in the context of two nucleic acid sequences makes reference to a specified degree of sequence identity between two sequences when aligned for maximum correspondence over a specified comparison window, as measured by sequence comparison algorithms or by visual inspection. The computer used to calculate the score may be any suitable computer, as described in further detail below.

In some embodiments, the score indicating the degree of sequence similarity may be a percentage identity between two sequences. As used herein, "percentage of sequence identity" means the value determined by comparing two optimally aligned sequences over a comparison window, wherein the portion of the polynucleotide sequence in the comparison window may include additions or deletions (i.e., gaps) as compared to the reference sequence (which does not include additions or deletions) for optimal alignment of the two sequences. The percentage is calculated by determining the number of positions at which the identical nucleic acid base occurs in both sequences to yield the number of matched positions, dividing the number of matched positions by the total number of positions in the window of comparison, and multiplying the result by 100 to yield the percentage of sequence identity.

Any suitable methods of alignment of sequences for comparison may be employed. Thus, the determination of percent identity between any two sequences can be accomplished using a mathematical algorithm. Preferred, non-limiting examples of such mathematical algorithms are the algorithm of Myers and Miller, CABIOS, 4:11 (1988); the local homology algorithm of Smith et al, Adv. Appl. Math., 2:482 (1981); the homology alignment algorithm of Needleman and Wunsch, JMB, 48:443 (1970); the search-for-similarity-method of Pearson and Lipman, Proc. Natl. Acad. Sci. USA, 85:2444 (1988); the algorithm of Karlin and Altschul, Proc. Natl. Acad. Sci. USA, 87:2264 (1990); modified as in Karlin and Altschul, Proc. Natl. Acad. Sci. USA, 90:5873 (1993).

In certain embodiments, the score indicating the degree of sequence similarity between each of the matched capture sequences and the sequence read is based on the length of sequence identity between the matched capture sequence and the sequence read, the string edit distance between the matched capture sequence and the sequence read, the position within the sequence read of each of the mismatches, or a combination thereof. Thus in certain embodiments, the score indicating the degree of sequence similarity between each of the matched capture sequences and the sequence read is a weighted score, wherein the score is based on a value representing the length of sequence identity between the matched capture sequence and the sequence read, and the value is weighted by the string edit distance between the matched capture sequence and the sequence read, the position within the sequence read of each of the mismatches, or a combination thereof. In some instances, the weighting includes subtracting the string edit distance from the length of sequence identity between the matched capture sequence and the sequence read. In some instances, the weighting involves weighting mismatches towards the beginning of the sequence read more heavily than mismatches towards the end of the sequence read.

Upon identifying a matched capture sequence with a calculated score above a threshold (**120**, **220**), the sequence read may be assigned to the capture sequence (**122**, **222**), as shown in Figs. 1 and 2. Thus, a further aspect of the present disclosure includes assigning the sequence read to the genomic location if the calculated score for a matched capture sequence is above a threshold. In certain embodiments, the assigning (**122**, **222**) involves associating the sequence read with the matched capture sequence with a calculated sequence similarity score above threshold in a data structure, e.g., a table, and writing the data structure in memory, e.g. a computer file in memory. In certain embodiments, a sequence read is assigned to all the matched capture sequences with a calculated sequence similarity score above threshold. In certain embodiments, a sequence read is assigned to a matched capture sequence whose calculated sequence similarity score is higher than the calculated sequence similarity score for all the other matched capture sequences of the sequence read. In certain embodiments, a sequence read is assigned to a matched capture sequence whose calculated sequence similarity score is above threshold and higher than the calculated sequence similarity score for all the other matched capture sequences of the sequence read.

In certain embodiments, a method of the present disclosure includes an assigning step that further includes identifying a matched capture sequence having the highest calculated score among all of the matched capture sequences as being the best match, and assigning the sequence read to the genomic location by adding the sequence read to a set of unique sequence reads matching the best matched capture sequence, wherein each unique sequence read in the set contains a subsequence identical to a subsequence of all the other sequence reads in the set. In such instances, the quality of the assignment may be represented by the highest calculated score among all the sequence reads belonging to the same set. In certain embodiments, the quality of the assignment may be represented by the average of the calculated scores of all the sequence reads belonging to the same set. In some embodiments, the subsequence identical to a subsequence of all the other sequence reads in the set may be a barcode sequence. Thus, sequence reads that are assigned to the same capture sequence and have the identical barcode sequence are likely to represent amplified fragments of DNA that were derived from the same fragmented genomic fragment. In certain embodiments, the method further includes counting the number of sets of unique sequence reads assigned to a capture sequence.

In certain embodiments, a sequence read is annotated as being not assigned to any genomic location or capture sequence if the calculated score for none of the matched capture sequence is above a threshold. In certain embodiments, a sequence read whose calculated score for none of the matched capture sequence is above a threshold is annotated to be further processed by an alternative method.

In some embodiments, the threshold against which the sequence similarity score of a matched capture sequence for a sequence read is compared is a predetermined, constant value. In certain embodiments, the threshold against which the sequence similarity score of a matched capture sequence for a sequence read is compared can be provided by the user. In certain embodiments, the threshold against which the sequence similarity score of a matched capture sequence for a sequence read is compared is determined based on the quality of the sequence read.

In certain embodiments, the method is performed on a plurality of sequence reads, thereby assigning a plurality of sequence reads to genomic locations. A plurality of sequence reads may be assigned sequentially, e.g., on a single processor, or a plurality of sequence reads may be assigned in parallel by the subject method, e.g., simultaneously on a plurality of processors, or a combination of both may occur. The method may be performed on all sequence reads (**102**, **202**) from a sequencing run, all sequence reads (**102**, **202**) from multiple sequencing runs, or a subset of the sequence reads (**102**, **202**) from one or more sequencing runs.

The above-described method can be implemented on a computer. In certain embodiments, a general-purpose computer can be configured to a functional arrangement for the methods and programs disclosed herein. The hardware architecture of such a computer is well known by a person skilled in the art, and can comprise hardware components including one or more processors (CPU), a random-access memory (RAM), a read-only memory (ROM), an internal or external data storage medium (e.g., hard disk drive). A computer system can also comprise one or more graphic boards for processing and outputting graphical information to display means. The above components can be suitably interconnected via a bus inside the computer. The computer can further comprise suitable interfaces for communicating with general-purpose external components such as a monitor, keyboard, mouse, network, etc. In some embodiments, the computer can be capable of parallel processing or can be part of a network configured for parallel or distributive computing to increase the processing power for the present methods and programs. In some embodiments, the program code read out from the storage medium can be written into a memory provided in an expanded board inserted in the computer, or an expanded unit connected to the computer, and a CPU or the like provided in the expanded board or expanded unit can actually perform a part or all of the operations according to the instructions of the program code, so as to accomplish the functions described below. In other embodiments, the method can be performed using a cloud computing system. In these embodiments, the data files and the programming can be exported to a cloud computer, which runs the program, and returns an output to the user.

A system can in certain embodiments comprise a computer that includes: a) a central processing unit; b) a main non-volatile storage drive, which can include one or more hard drives, for storing software and data, where the storage drive is controlled by disk controller; c) a system memory, e.g., high speed random-access memory (RAM), for storing system control programs, data, and application programs, including programs and data loaded from non-volatile storage drive; system memory can also include read-only memory (ROM); d) a user interface, including one or more input or output devices, such as a mouse, a keypad, and a display; e) an optional network interface card for connecting to any wired or wireless communication network, e.g., a printer; and f) an internal bus for interconnecting the aforementioned elements of the system.

The memory of a computer system can be any device that can store information for retrieval by a processor, and can include magnetic or optical devices, or solid state memory devices (such as volatile or non-volatile RAM). A memory or memory unit can have more than one physical memory device of the same or different types (for example, a memory can have multiple memory devices such as multiple drives, cards, or multiple solid state memory devices or some combination of the same). With respect to computer readable media, "permanent memory" refers to memory that is permanent. Permanent memory is not erased by termination of the electrical supply to a computer or processor. Computer hard-drive ROM (i.e., ROM not used as virtual memory), CD-ROM, floppy disk and DVD are all examples of permanent memory. Random Access Memory (RAM) is an example of non-permanent (i.e., volatile) memory. A file in permanent memory can be editable and re-writable.

Operation of the computer is controlled primarily by an operating system, which is executed by the central processing unit. The operating system can be stored in a system memory. In some embodiments, the operating system includes a file system. In addition to an operating system, one possible implementation of the system memory includes a variety of programming files and data files for implementing the method described below. In certain cases, the programming can contain a program, where the program can be composed of various modules, and a user interface module that permits a user to manually select or change the inputs to or the parameters used by the program. The data files can include various inputs for the program.

In certain embodiments, instructions in accordance with the method described herein can be coded onto a computer-readable medium in the form of "programming," where the term "computer readable medium" as used herein refers to any storage or transmission medium that participates in providing instructions and/or data to a computer for execution and/or processing. Examples of storage media include a floppy disk, hard disk, optical disk, magneto-optical disk, CD-ROM, CD-R, magnetic tape, non-volatile memory card, ROM, DVD-ROM, Blue-ray disk, solid state disk, and network attached storage (NAS), whether or not such devices are internal or external to the computer. A file containing information can be "stored" on computer readable medium, where "storing" means recording information such that it is accessible and retrievable at a later date by a computer.

The computer-implemented method described herein can be executed using programs that can be written in one or more of any number of computer programming languages. Such languages include, for example, Java (Sun Microsystems, Inc., Santa Clara, CA), Visual Basic (Microsoft Corp., Redmond, WA), and C++ (AT&T Corp., Bedminster, NJ), as well as any many others.

In any embodiment, data can be forwarded to a "remote location," where "remote location," means a location other than the location at which the program is executed. For example, a remote location could be another location (e.g., office, lab, etc.) in the same city, another location in a different city, another location in a different state, another location in a different country, etc. As such, when one item is indicated as being "remote" from another, what is meant is that the two items can be in the same room but separated, or at least in different rooms or different buildings, and can be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. Examples of communicating media include radio or infra-red transmission channels as well as a network connection to another computer or networked device, and the internet or including email transmissions and information recorded on websites and the like.

Some embodiments include implementation on a single computer, or across a network of computers, or across networks of networks of computers, for example, across a network cloud, across a local area network, on hand-held computer devices, etc. In certain embodiments, one or more of the steps described herein are implemented on a computer program(s). Such computer programs execute one or more of the steps described herein. In some embodiments, implementations of the subject method include various data structures, categories, and modifiers described herein, encoded on computer-readable medium(s) and transmissible over communications network(s).

Software, web, Internet, cloud, or other storage and computer network implementations of the present invention could be accomplished with standard programming techniques to accomplish the various assigning, calculating, identifying, scoring, accessing, generating or discarding steps.

In certain embodiments, a computer-implemented method of the present disclosure is coded into a sequence read assignment program, as described above. Thus, provided herein is a method for assigning a sequence read to a genomic location, the method involving inputting a set of capture sequences used to enrich a nucleic acid sample by hybridization to a plurality of capture sequences in the set into a computer system containing a sequence read assignment program, as described above, wherein the sequence read assignment program includes instructions for accessing a file containing a sequence read, wherein the sequence read is obtained from the enriched nucleic acid sample, and assigning the sequence read to a genomic location by identifying a capture sequence as being a match with the sequence read if the sequence read contains one or more subsequences of the capture sequence, calculating, using a computer, a score indicating the degree of sequence similarity between each of the matched capture sequences and the sequence read, and assigning the sequence read to the genomic location if the calculated score for a matched capture sequence is above a threshold, inputting a file containing the sequence read into the sequence read assignment program, and executing the sequence read assignment program. In some embodiments, the sequence read assignment program may be executed on a local computer that also stores the files containing the sequence reads (**102**, **202**) and the capture sequences. In certain embodiments, the sequence read assignment program may be executed at a remote location, e.g., a remote server, and the files containing the sequence reads (**102**, **202**) and the capture sequences may also be stored at one or more remote locations. In such instances, the inputting may involve inputting on a local computer the one or more remote locations where the file containing the sequence reads (**102**, **202**) and the capture sequences are stored and transmitting the location information to the remote location where the sequence read assignment program is executed.

One implementation of the subject method is described below. The target enrichment kit is defined by a set of capture probes that are selectively hybridizing to DNA fragments located in the targeted regions of the genome. Only the captured DNA fragments are amplified and can yield sequencing reads.

The method of assigning a sequence read to a genomic region includes:
- Reading all capture sequences from probes of the target enrichment design and the genomic location that they were designed to match.
- Building several fast access tables of these probes, keyed by short DNA subsequences that matching reads captured by those probes need to exhibit. The different tables are using different subsequences of the capture region so that if a DNA fragment has a sequence slightly different (genomic variant) from the reference sequence that the capture region was designed for, some of the keys computed from the read will not match but others will.
- For each read (or read pair) from the sequencing run:
   i) Extract a set of short of subsequences keys expected to match one or several of the probes in the relevant tables.
   ii) Score the quality of the match between the read and each of the probes by evaluating the biochemical effectiveness of the match. For instance, if the match between the read and the capture zone of the probe decreases as the number of mismatched bases increases, and mismatches towards the beginning of the read have a more pronounced effect, the score could be the length of the matching sequence minus the string edit distance between the read and the probe matching zones, plus the index of the first mismatch (or the full length of the sequence if no mismatch).
   iii) Tag the read with all the probe IDs that have a scoring match above a given threshold if any.
   iv) If no probe matches above the threshold, apply extended matching processing. This is a set of algorithms that are not expected to be called often and can use expensive (in time and memory) methods to try to model very infrequent matching situations, like unlikely (but possible) read errors and multiprobe matching if the biology allows.
   v) If absolutely not matching can be recognized write in a separate output file as read to be further processed by other means.
- At this point either:
   i) Write the tagged read to an output file or stream; or
   ii) add to the set of unique reads matching the best probe match. By unique it is meant the set of read sequence (or pair of sequences) and molecular bar code sequence if any is unique. If the set already has an entry for the set of reads and barcode, merge the quality values of the two reads so as to store the best quality seen for that particular set of sequences.
- If the reads have been stored by probe sequence, after processing a large set of reads or all the reads (typically, stop to unload memory if memory is full) write the set of unique reads/barcode per probe ID to an output file or pass the data structures to another module for further processing, for instance variant calling.
- If processing the data generated several intermediate files at the previous step, merge the previous files by probe ID and write the global reads per probe file. For an engineering grade computer (16GB of RAM of more) a very large data set can be matched to the largest set of probes in one pass without having to write intermediate files than need to be merged afterwards.

### UTILITY

The methods and software implementations of the methods disclosed herein find use in various sequence analysis applications, e.g., mapping sequence reads obtained from an enriched genomic sample to a reference genome. Because the methods disclosed herein take advantage of the additional information available from the design of the target enrichment panel used to produce the sequence reads, the computing time of the genomic location tagging may be reduced by about a factor of 10, and tagging to locations that are incompatible with the experimental design, which may otherwise occur to 2-3% of the sequence reads, is avoided.

The assigned sequence reads may be used to assemble a plurality of discrete sequence that each corresponds to a unique genomic fragment, including any potential variants, using a variety of different methods. Assigned sequence reads may be assembled using any suitable method, basic steps of which are described in a variety of publications such as Myers et al (Science 2000 287: 2196-204), Batzoglou et al (Genome Research 2002 12: 177-89), Dohm et al (Genome Research 2007 17 : 1697-706) and Boisvert et al (Journal of Computational Biology 2010 17: 1519-33), which all disclose such methods. For each enriched region, the assigned sequence reads can be assembled to produce a single pile-up that is examined to identify sequence reads that have a nucleotide variation (e.g., a substitution, insertion or deletion) at a particular position. Assigned sequence reads can also be assembled by aligning each read to a reference sequence, such as a reference genome.

The method of the present disclosure may be used on sequence reads derived from a biological sample, e.g., a biopsy, obtained from a patient. For example, the method may be employed as part of a sequencing protocol pipeline to identify and/or estimate the amount of mutant copies of a genomic locus that are in a biological sample that contains both wild type copies of a genomic locus and mutant copies of the genomic locus that have a point mutation relative to the wild type copies of the genomic locus. In this example, the sample may contain at least 100 times (e.g., at least 1,000 times, at least 5,000 times, at least 10,000 times, at least 50,000 times or at least 100,000 times) more wild type copies of the genomic locus than mutant copies of the genomic locus.

The method may also find use in mapping sequence reads from nucleic acids enriched to detect an oncogenic mutation (which may be a somatic mutation) in, e.g., PIK3CA, NRAS, KRAS, JAK2, HRAS, FGFR3, FGFR1, EGFR, CDK4, BRAF, RET, PGDFRA, KIT or ERBB2, which mutation may be associated with breast cancer, melanoma, renal cancer, endometrial cancer, ovarian cancer, pancreatic cancer, leukemia, colorectal cancer, prostate cancer, mesothelioma, glioma, medullobastoma, polycythemia, lymphoma, sarcoma or multiple myeloma (see, e.g., Chial Proto-oncogenes to oncogenes to cancer. Nature Education 2008 1: 1).

The citation of any publication is for its disclosure prior to the filing date and should not be construed as an admission that the present invention is not entitled to antedate such publication by virtue of prior invention.

## Claims

1. A computer-implemented method for assigning a sequence read to a genomic location, comprising:
a) accessing a file comprising a sequence read, wherein the sequence read is obtained from a nucleic acid sample that comprises amplified copies of fragmented genomic nucleic acids that have been enriched by hybridization to a plurality of capture sequences that target multiple genomic locations of interest, wherein the fragmented genomic nucleic acids are fragmented by enzymatically cleaving genomic nucleic acids at predetermined sites; and
b) assigning the sequence read to a genomic location by:
i) identifying a capture sequence as being a match with the sequence read if the sequence read comprises one or more subsequences of the capture sequence;
ii) calculating, using a computer, a score indicating the degree of sequence similarity between each of the matched capture sequences and the sequence read; and
iii) assigning the sequence read to the genomic location if the calculated score for a matched capture sequence is above a threshold.

2. The method according to claim 1, wherein:
the identifying step i) comprises identifying one or more of the capture sequences as being a match with the sequence read if a terminal region of the sequence read comprises one or more subsequences of the capture sequences, wherein the terminal region is optionally in the range of 10 bp (base pairs) to 50 bp from an end of the sequence read; and/or
the identifying step i) further comprises generating a data structure, wherein the capture sequences are stored in the data structure as values mapped by sequence keys comprising subsequences of the capture sequences, and the identifying step comprises identifying one or more of the capture sequences as being a match with the sequence read if the sequence read comprises one or more sequence keys.

3. The method according to claim 1 or 2, wherein:
the one or more subsequences are in the range of 5 bp to 15 bp in length; and/or
the one or more subsequences of the capture sequence is selected from between 4 to 20 subsequences of the capture sequence, wherein the subsequences optionally are tiled across the entire capture sequence.

4. The method according to any one of claims 1-3, wherein the calculated score is calculated based on the length of sequence identity between the matched capture sequence and the sequence read, the string edit distance between the matched capture sequence and the sequence read, the position within the sequence read of each of the mismatches, or a combination thereof.

5. The method according to any one of claims 1-4, wherein the sequence read is a paired-end sequence read.

6. The method according to any one of claims 1-5, wherein the capture sequences hybridize to an end of the nucleic acids.

7. The method according to any one of claims 1-6, wherein the assigning step b) further comprises discarding a sequence read if the sequence read does not comprise any subsequences of the capture sequences.

8. The method according to any one of claims 1-7, wherein the method is performed on a plurality of sequence reads, thereby assigning a plurality of sequence reads to genomic locations.

9. The method according to any one of claims 1-8, wherein the assigning step b) further comprises:
iv) identifying a matched capture sequence having the highest calculated score among all of the matched capture sequences as being the best match; and
v) assigning the sequence read to the genomic location by adding the sequence read to a set of unique sequence reads matching the best matched capture sequence, wherein each unique sequence read in the set comprises a subsequence identical to a subsequence of all the other sequence reads in the set.

10. The method according to claim 9, wherein the subsequence identical to a subsequence of all the other sequence reads in the set is a barcode sequence, wherein the method optionally further comprises counting the number of sets of unique sequence reads assigned to a capture sequence.

11. The method according to any one of claims 1-10, wherein the capture sequences comprises from 10² to 10⁸ distinct sequences.

12. A method for assigning a sequence read to a genomic location, comprising:
a) inputting a set of capture sequences used to enrich a nucleic acid sample by hybridization to a plurality of capture sequences in the set into a computer system comprising a sequence read assignment program, wherein the sequence read assignment program comprises the instructions of a) to b) of claim 1,
b) inputting a file comprising the sequence read into the sequence read assignment program; and
c) executing the sequence read assignment program.

13. A computer readable storage medium comprising a sequence read assignment program comprising instructions for:
a) accessing a file comprising a sequence read, wherein the sequence read is obtained from a nucleic acid sample that comprises amplified copies of fragmented genomic nucleic acids that have been enriched by hybridization to a plurality of capture sequences that target multiple genomic locations of interest, wherein the fragmented genomic nucleic acids are fragmented by enzymatically cleaving genomic nucleic acids at predetermined sites; and
b) assigning the sequence read to a genomic location by:
i) identifying a capture sequence as being a match with the sequence read if the sequence read comprises one or more subsequences of the capture sequence;
ii) calculating, using a computer, a score indicating the degree of sequence similarity between each of the matched capture sequences and the sequence read; and
iii) assigning the sequence read to the genomic location if the calculated score for a matched capture sequence is above a threshold,
wherein the identifying step i) comprises generating a data structure, wherein the capture sequences are stored in the data structure as values mapped by sequence keys comprising subsequences of the capture sequences, and the identifying step comprises identifying one or more of the capture sequences as being a match with the sequence read if the sequence read comprises one or more sequence keys.

## Patentansprüche

1. Computer-implementiertes Verfahren zum Zuordnen eines Sequenz-Reads zu einer genomischen Stelle, umfassend:
a) Zugreifen auf eine Datei, die einen Sequenz-Read umfasst, wobei der Sequenz-Read von einer Nukleinsäureprobe erhalten wurde, die amplifizierte Kopien von fragmentierten genomischen Nukleinsäuren umfasst, die durch Hybridisierung mit einer Mehrzahl von Einfangsequenzen angereichert wurden, die auf mehrere genomische Stellen von Interesse abzielen, wobei die fragmentierten genomischen Nukleinsäuren durch enzymatische Spaltung genomischer Nukleinsäuren an vorbestimmten Stellen fragmentiert werden; und
b) Zuordnen des Sequenz-Reads zu einer genomischen Stelle durch:
i) Identifizieren einer Fangsequenz als übereinstimmend mit dem Sequenz-Read, wenn der Sequenz-Read eine oder mehrere Teilsequenzen der Fangsequenz umfasst;
ii) Berechnen, unter Verwendung eines Computers, einer Bewertungszahl, die den Grad der Sequenzähnlichkeit zwischen jeder der übereinstimmenden Fangsequenzen und dem Sequenz-Read angibt; und
iii) Zuordnen des Sequenz-Reads zu der genomischen Stelle, wenn die berechnete Bewertungszahl für eine übereinstimmende Fangsequenz über einem Schwellenwert liegt.

2. Verfahren nach Anspruch 1, wobei:
der Identifizierungsschritt i) das Identifizieren einer oder mehrerer der Fangsequenzen als übereinstimmend mit dem Sequenz-Read umfasst, wenn eine terminale Region des Sequenz-Reads eine oder mehrere Teilsequenzen der Fangsequenzen umfasst, wobei die terminale Region optional im Bereich von 10 bp (Basenpaaren) bis 50 bp von einem Ende des Sequenz-Reads liegt; und/oder
der Identifizierungsschritt i) ferner das Erzeugen einer Datenstruktur umfasst, wobei die Fangsequenzen in der Datenstruktur als Werte gespeichert sind, die durch Sequenzschlüssel abgebildet werden, die Teilsequenzen der Fangsequenzen umfassen, und der Identifizierungsschritt das Identifizieren einer oder mehrerer der Fangsequenzen als Übereinstimmung mit dem Sequenz-Read umfasst, wenn der Sequenz-Read einen oder mehrere Sequenzschlüssel umfasst.

3. Verfahren nach Anspruch 1 oder 2, wobei:
die eine oder mehreren Teilsequenzen eine Länge im Bereich von 5 bp bis 15 bp aufweisen; und/oder
die eine oder die mehreren Teilsequenzen der Fangsequenz aus 4 bis 20 Teilsequenzen der Fangsequenz ausgewählt werden, wobei die Teilsequenzen optional über die gesamte Fangsequenz verschachtelt sind.

4. Verfahren nach einem der Ansprüche 1 bis 3, wobei die berechnete Bewertungszahl auf der Grundlage der Länge der Sequenzidentität zwischen der übereinstimmenden Fangsequenz und dem Sequenz-Read, der String-Edit-Distanz zwischen der übereinstimmenden Fangsequenz und dem Sequenz-Read, der Position innerhalb des Sequenz-Reads von jeder der Fehlpaarungen oder einer Kombination davon berechnet wird.

5. Verfahren nach einem der Ansprüche 1-4, wobei der Sequenz-Read ein Paired-End-Sequenz-Read ist.

6. Verfahren nach einem der Ansprüche 1-5, wobei die Fangsequenzen an ein Ende der Nukleinsäuren hybridisieren.

7. Verfahren nach einem der Ansprüche 1-6, wobei der Zuordnungsschritt b) ferner das Verwerfen eines Sequenz-Reads umfasst, wenn der Sequenz-Read keine Teilsequenzen der Fangsequenzen umfasst.

8. Verfahren nach einem der Ansprüche 1-7, wobei das Verfahren an einer Mehrzahl von Sequenz-Reads durchgeführt wird, wodurch eine Mehrzahl von Sequenz-Reads genomischen Stellen zugewiesen wird.

9. Verfahren nach einem der Ansprüche 1 bis 8, wobei der Zuordnungsschritt b) ferner umfasst:
iv) Identifizieren einer übereinstimmenden Fangsequenz mit der höchsten berechneten Bewertungszahl unter allen übereinstimmenden Fangsequenzen als die beste Übereinstimmung; und
v) Zuordnen des Sequenz-Reads zu der genomischen Stelle durch Hinzufügen des Sequenz-Reads zu einem Satz von einzigartigen Sequenz-Reads, die mit der am besten übereinstimmenden Fangsequenz übereinstimmen, wobei jeder einzigartige Sequenz-Read in dem Satz eine Teilsequenz umfasst, die mit einer Teilsequenz aller anderen Sequenz-Reads in dem Satz identisch ist.

10. Verfahren nach Anspruch 9, wobei die Teilsequenz, die mit einer Teilsequenz aller anderen Sequenz-Reads in dem Satz identisch ist, eine Barcode-Sequenz ist, wobei das Verfahren optional ferner das Zählen der Anzahl der Sätze einzigartiger Sequenz-Reads umfasst, die einer Fangsequenz zugeordnet sind.

11. Verfahren nach einem der Ansprüche 1-10, wobei die Fangsequenzen 102 bis 108 verschiedene Sequenzen umfassen.

12. Verfahren zum Zuordnen eines Sequenz-Reads zu einer genomischen Stelle, umfassend:
a) Eingeben eines Satzes von Fangsequenzen, die zur Anreicherung einer Nukleinsäureprobe durch Hybridisierung mit einer Mehrzahl von Fangsequenzen in dem Satz verwendet wurden, in ein Computersystem, das ein Sequenz-Read-Zuordnungsprogramm umfasst, wobei das Sequenz-Read-Zuordnungsprogramm die Anweisungen von a) bis b) von Anspruch 1 umfasst,
b) Eingeben einer Datei mit dem Sequenz-Read in das Sequenz-Read-Zuordnungsprogramm; und
c) Ausführen des Sequenz-Read-Zuordnungsprogramms.

13. Computerlesbares Speichermedium mit einem Sequenz-Read-Zuweisungsprogramm, das Anweisungen enthält für:
a) Zugreifen auf eine Datei, die einen Sequenz-Read umfasst, wobei der Sequenz-Read von einer Nukleinsäureprobe erhalten wurde, die amplifizierte Kopien von fragmentierten genomischen Nukleinsäuren umfasst, die durch Hybridisierung mit einer Mehrzahl von Fangsequenzen angereichert wurden, die auf mehrere genomische Stellen von Interesse abzielen, wobei die fragmentierten genomischen Nukleinsäuren durch enzymatische Spaltung genomischer Nukleinsäuren an vorbestimmten Stellen fragmentiert werden; und
b) Zuordnen des Sequenz-Reads zu einer genomischen Stelle durch:
i) Identifizieren einer Fangsequenz als übereinstimmend mit dem Sequenz-Read, wenn der Sequenz-Read eine oder mehrere Teilsequenzen der Fangsequenz umfasst;
ii) Berechnen, unter Verwendung eines Computers, einer Bewertungszahl, die den Grad der Sequenzähnlichkeit zwischen jeder der übereinstimmenden Fangsequenzen und dem Sequenz-Read angibt; und
iii) Zuordnen des Sequenz-Reads zu der genomischen Stelle, wenn die berechnete Bewertungszahl für eine übereinstimmende Fangsequenz über einem Schwellenwert liegt,
wobei der Identifizierungsschritt i) das Erzeugen einer Datenstruktur umfasst, wobei die Fangsequenzen in der Datenstruktur als Werte gespeichert sind, die durch Sequenzschlüssel abgebildet werden, die Teilsequenzen der Fangsequenzen umfassen, und der Identifizierungsschritt das Identifizieren einer oder mehrerer der Fangsequenzen als eine Übereinstimmung mit dem Sequenz-Read umfasst, wenn der Sequenz-Read einen oder mehrere Sequenzschlüssel umfasst.

## Revendications

1. Procédé mis en oeuvre par un ordinateur pour attribuer une lecture de séquence à une position génomique, comprenant :
a) l'accès à un fichier comprenant une lecture de séquence, dans lequel la lecture de séquence est obtenue à partir d'un échantillon d'acide nucléique qui comprend des copies amplifiées d'acides nucléiques génomiques fragmentés qui ont été enrichis par hybridation avec une pluralité de séquences de capture qui ciblent de multiples positions génomiques d'intérêt, dans lequel les acides nucléiques génomiques fragmentés sont fragmentés par clivage enzymatique d'acides nucléiques génomiques à des sites prédéterminés ; et
b) l'attribution de la lecture de séquence à une position génomique par :
i) l'identification d'une séquence de capture comme concordant avec la lecture de séquence si la lecture de séquence comprend une ou plusieurs sous-séquences de la séquence de capture ;
ii) le calcul, à l'aide d'un ordinateur, d'un score indiquant le degré de similarité de séquence entre chacune des séquences de capture concordantes et la lecture de séquence ; et
iii) l'attribution de la lecture de séquence à la position génomique si le score calculé pour une séquence de capture concordante est supérieur à un seuil.

2. Procédé selon la revendication 1, dans lequel :
l'étape d'identification i) comprend l'identification d'une ou plusieurs desdites séquences de capture comme concordant avec la lecture de séquence si une région terminale de la lecture de séquence comprend une ou plusieurs sous-séquences des séquences de capture, dans lequel la région terminale est facultativement comprise dans la plage de 10 bp à 50 pb (paires de bases) à partir d'une extrémité de la lecture de séquence ; et/ou
l'étape d'identification i) comprend en outre la génération d'une structure de données, dans lequel les séquences de capture sont stockées dans la structure de données en tant que valeurs mappées par des clés de séquence comprenant des sous-séquences des séquences de capture, et l'étape d'identification comprend l'identification d'une ou plusieurs des séquences de capture comme étant une concordance avec la lecture de séquence si la lecture de séquence comprend une ou plusieurs clés de séquence.

3. Procédé selon la revendication 1 ou 2, dans lequel :
lesdites une ou plusieurs sous-séquences ont une longueur comprise dans la plage de 5 pb à 15 pb ; et/ou
lesdites une ou plusieurs sous-séquences de la séquence de capture sont sélectionnées parmi 4 à 20 sous-séquences de la séquence de capture, dans lequel les sous-séquences sont facultativement disposées en mosaïque sur toute la séquence de capture.

4. Procédé selon l'une quelconque des revendications 1 à 3, dans lequel le score calculé est calculé sur la base de la longueur de l'identité de séquence entre la séquence de capture concordante et la lecture de séquence, de la distance d'édition de chaîne entre la séquence de capture concordante et la lecture de séquence, de la position dans la lecture de séquence de chacune des discordances, ou d'une combinaison de celles-ci.

5. Procédé selon l'une quelconque des revendications 1 à 4, dans lequel la lecture de séquence est une lecture de séquence à extrémité appariée.

6. Procédé selon l'une quelconque des revendications 1 à 5, dans lequel les séquences de capture s'hybrident à une extrémité des acides nucléiques.

7. Procédé selon l'une quelconque des revendications 1 à 6, dans lequel l'étape d'attribution b) comprend en outre le rejet d'une lecture de séquence si la lecture de séquence ne comprend aucune sous-séquence des séquences de capture.

8. Procédé selon l'une quelconque des revendications 1 à 7, dans lequel le procédé est mis en oeuvre sur une pluralité de lectures de séquence, attribuant ainsi une pluralité de lectures de séquence à des positions génomiques.

9. Procédé selon l'une quelconque des revendications 1 à 8, dans lequel l'étape d'attribution b) comprend en outre :
iv) l'identification d'une séquence de capture concordante ayant le score calculé le plus élevé parmi toutes les séquences de capture concordantes comme étant la meilleure concordance ; et
v) l'attribution de la lecture de séquence à la position génomique en ajoutant la lecture de séquence à un ensemble de lectures de séquence uniques concordant avec la séquence de capture la plus concordante, dans lequel chaque lecture de séquence unique dans l'ensemble comprend une sous-séquence identique à une sous-séquence de toutes les autres lectures de séquence dans l'ensemble.

10. Procédé selon la revendication 9, dans lequel la sous-séquence identique à une sous-séquence de toutes les autres lectures de séquence dans l'ensemble est une séquence de code-barres, dans lequel le procédé comprend en outre facultativement le comptage du nombre d'ensembles de lectures de séquence uniques attribuées à une séquence de capture.

11. Procédé selon l'une quelconque des revendications 1 à 10, dans lequel les séquences de capture comprennent de 10² à 10⁸ séquences distinctes.

12. Procédé pour attribuer une lecture de séquence à une position génomique, comprenant :
a) l'entrée, dans un système informatique comprenant un programme d'attribution de lecture de séquence, d'un ensemble de séquences de capture utilisées pour enrichir un échantillon d'acide nucléique par hybridation avec une pluralité de séquences de capture dans l'ensemble, dans lequel le programme d'attribution de lecture de séquence comprend les instructions a) et b) de la revendication 1,
b) l'entrée d'un fichier comprenant la lecture de séquence dans le programme d'attribution de lecture de séquence ; et
c) l'exécution du programme d'attribution de lecture de séquence.

13. Support de stockage lisible par ordinateur comprenant un programme d'attribution de lecture de séquence comprenant des instructions pour :
a) l'accès à un fichier comprenant une lecture de séquence, dans lequel la lecture de séquence est obtenue à partir d'un échantillon d'acide nucléique qui comprend des copies amplifiées d'acides nucléiques génomiques fragmentés qui ont été enrichis par hybridation avec une pluralité de séquences de capture qui ciblent de multiples positions génomiques d'intérêt, dans lequel les acides nucléiques génomiques fragmentés sont fragmentés par clivage enzymatique d'acides nucléiques génomiques à des sites prédéterminés ; et
b) l'attribution de la lecture de séquence à une position génomique par :
i) l'identification d'une séquence de capture comme concordant avec la lecture de séquence si la lecture de séquence comprend une ou plusieurs sous-séquences de la séquence de capture ;
ii) le calcul, à l'aide d'un ordinateur, d'un score indiquant le degré de similarité de séquence entre chacune des séquences de capture concordantes et la lecture de séquence ; et
iii) l'attribution de la lecture de séquence à la position génomique si le score calculé pour une séquence de capture concordante est supérieur à un seuil,
dans lequel l'étape d'identification i) comprend la génération d'une structure de données, dans lequel les séquences de capture sont stockées dans la structure de données en tant que valeurs mappées par des clés de séquence comprenant des sous-séquences des séquences de capture, et l'étape d'identification comprend l'identification d'une ou plusieurs des séquences de capture comme étant une concordance avec la lecture de séquence si la lecture de séquence comprend une ou plusieurs clés de séquence.
